Europäisches Patentamt

European Patent Office

Office européen des brevets

(19)

(11) Veröffentlichungsnummer: **0 337 057 B1**

(12) **EUROPÄISCHE PATENTSCHRIFT**

(45) Veröffentlichungstag der Patentschrift: **21.04.93**

(51) Int. Cl.5: **G01N 33/68**, G01N 33/574, G01N 33/577, C12P 21/00

(21) Anmeldenummer: **89101074.6**

(22) Anmeldetag: **23.01.89**

(54) **Verfahren zur Identifizierung des Ursprungs einer Zell- oder Gewebsprobe.**

(30) Priorität: **26.01.88 DE 3802093**
**10.05.88 DE 3815932**

(43) Veröffentlichungstag der Anmeldung:
**18.10.89 Patentblatt 89/42**

(45) Bekanntmachung des Hinweises auf die Patenterteilung:
**21.04.93 Patentblatt 93/16**

(84) Benannte Vertragsstaaten:
**AT BE CH DE ES FR GB GR IT LI LU NL SE**

(56) Entgegenhaltungen:
**EP-A- 0 163 304**
**EP-A- 0 267 355**
**EP-A- 0 267 356**
**WO-A-85/03132**

**CHEMICAL ABSTACTS, Band 95, 1981, Columbus, OH (US); W.J.NELSON et al., Seite 302, Nr. 37953a***

**JOURNAL OF IMMUNOLOGICAL METHODS, Band 85, 1985, Elsevier Science Publishers B.V., Amsterdam (NL); G.K.HANSSON, Seiten 401-407***

**CHEMICAL ABSTRACTS, Band 96, 1982, Columbus, OH (US); I.VIRTANEN et al., Seite 498, Nr. 159850f***

**CHEMICAL ABSTRACTS, Band 96, 1982, Columbus, OH (US); P.TRAUB et al., Seite 458, Nr. 101416c***

(73) Patentinhaber: **PROGEN Biotechnik GmbH**
**Im Neuenheimer Feld 519**
**W-6900 Heidelberg(DE)**

(72) Erfinder: **Bruder, Gerda, Dr.**
**Bergheimer Str. 110a**
**W-6900 Heidelberg(DE)**
Erfinder: **Franke, Werner Wilhelm, Dr.**
**Landfriedstr. 6**
**W-6900 Heidelberg(DE)**

(74) Vertreter: **Hach, Hans Karl, Dr.**
**Patentanwaltskanzlei Müller, Clemens & Hach, Lerchenstrasse 56**
**W-7100 Heilbronn (DE)**

EP 0 337 057 B1

Rank Xerox (UK) Business Services
(3.10/3.5x/3.0.1)

EP 0 337 057 B1

**Beschreibung**

Die Erfindung betrifft ein Verfahren zur Identifizierung des Differenzierungszustandes und des Ursprungs einer Zellgewebsprobe oder von Zellgewebstrümmern in Körperflüssigkeit durch Solubilisierung der in dieser Probe beziehungsweise Körperflüssigkeit vorhandenen unlöslichen Intermediärfilamentproteine in Form von löslichen Fragmenten, vorzugsweise der alpha-helikalen Mittelstücke, und Identifizierung dieser so erhaltenen oder bereits ohne zusätzliche Aufbereitung in der Probe enthaltenen Fragmente nach ihrem Ursprungsgewebetyp durch Immunreaktion mit spezifischen Antikörpern, wobei als Antikörper solche eingesetzt werden, die zur Identifizierung - vorzugsweise zur eindeutigen Identifizierung - von Intermediärfilamentproteinen nach ihrem Ursprungsgewebetyp geeignet sind und außerdem nach mehreren verschiedenen bekannten Testverfahren mit dem alpha-helikalen Mittelstück - beziehungsweise einem Epitop-tragenden Fragment davon - eines durch Rekonstitution aus gereinigten Monomeren des jeweils betreffenden Intermediärfilamentproteins gewonnenen Standards immunologisch reagieren.

Aus der als Stand der Technik geltenden europäischen Anmeldung 87 10 4557.1 (EP-A-0 267 355) ist ein Verfahren bekannt, bei dem zur Identifizierung gewebestypischer, unlöslicher Cytoskelettproteine, die in Lösung gebrachten Fragmente derselben immunologisch bestimmt und quantifiziert werden. Die Identifikation dient zur Auffindung von Gewebsläsionen oder anderen pathologischen Herden und Metastasen in lebenden Organismen.

Bei der zum Stande der Technik gehörigen europäischen Anmeldung 87 10 4558.9 (EP-A-0 267 356) werden zum Auffinden pathologischer Herde in lebenden Organismen die durch die ins Serum gelangenden Cytoskelettproteine generierten, zugeordneten Antikörper immunologisch identifiziert und quantifiziert.

Aufgabe der Erfindung ist es, mit dem eingangs beschriebenen Verfahren, quantifizierbare Ergebnisse zu gewinnen. Die Erfindung löst diese Aufgabe dadurch, daß ein Standard aus den zu identifizierenden Mittelstücken beziehungsweise Epitoptragenden Fragmenten hergestellt wird, indem aus dem entsprechenden Gewebe oder einer geeigneten Zellkultur-Linie durch Extraktion und Reinigung die einzelnen Cytoskelett-Polypeptide isoliert werden, daß dann aus diesen Polypeptiden beziehungsweise im Falle der Cytokeratine aus einem äquimolaren Gemisch von Polypeptiden der relativ basischen (Typ II) und der sauren (Typ I) Unterfamilien als nächste Untereinheiten Dimere, sodann Tetramere und schließlich Protofilamente gebildet werden, und daß dann durch kontrollierte Verdauung mit Protease die alpha-helikalen Mittelstücke dieser Intermediärfilamente als dimere oder tetramere Komplexe freigesetzt, isoliert und gereinigt und als Standard eingesetzt werden.

Die Solubilisierung der zu identifizierenden Intermediärfilamentproteine erfolgt vorzugsweise bis maximal zur einfachen Teilung der alpha-helikalen Mittelstücke in jeweils zwei Mittelstückfragmente und dadurch, daß solche Antikörper eingesetzt werden, deren Epitope durch die einfache Teilung unbeeinflußt an mindestens einem dieser Mittelstückfragmente sitzen und daß zur Quantifizierung und Identifizierung der alpha-helikalen Mittelstücke die Zahl der diese Epitope aufweisenden Mittelstückfragmente bestimmt wird.

Dabei verfährt man am besten derart, daß die Solubilisierung mit Protease im Enzym-zu-Substrat-Verhältnis und einer Abbauzeit erfolgt, die derart bemessen ist, daß von allen zu identifizierenden Intermediärfilamentproteinen die alphahelikalen Mittelstücke beziehungsweise Epitop-tragenden Fragmente davon freigesetzt sind.

Vorzugsweise macht man sich den Umstand zunutze, daß aufgrund der Vorarbeiten und Praktizierung der eingangs erwähnten bekannten histologischen Identifizierung geeignete Antikörper bekannt sind, die zur Identifizierung von IF-Proteinen geeignet sind. Aus diesen Antikörpern können dann durch die gekennzeichneten Maßnahmen solche ausgewählt werden, die für die serologische Identifizierung geeignet sind, und das sind solche, deren Epitop auf dem jeweiligen alpha-helikalen Mittelstück sitzt, das gegenüber weiteren proteolytischen und anderen Abbau-Prozessen relativ stabil ist.

So können zum Beispiel für die häufig vorkommenden Cytokeratine 8, 18 und 19 die folgenden, im Handel erhältlichen Antikörper verwendet werden: CAM 5.2, erhältlich von Becton Dickinson, Mtn. View, CA, USA; $K_G8.13$, erhältlich von BioMakor, Rehovot, Israel; $K_s$pan 1-8, erhältlich von PROGEN, Heidelberg, Bundesrepublik Deutschland, und hinterlegt bei der ECACC = EUROPEAN COLLECTION OF ANIMAL CELL CULTURES (PHLS CAMR, Porton Down, Salisbury, Wilts, Großbritannien) unter der Nr. 88011901 (siehe nachfolgende Tabelle 2); CK-2, erhältlich von Boehringer Mannheim, Bundesrepublik Deutschland; $K_s19.2$, erhältlich von PROGEN, Heidelberg, Bundesrepublik Deutschland und hinterlegt bei der ECACC unter der Hinterlegungsnummer 87111302. Antikörper $K_s19.2$ ist spezifisch für Cytokeratin 19 (siehe nachfolgende Tabelle 3).

Weitere Antikörper, die bei der Anwendung der Erfindung verwendet werden können, sind $K_s8-17.2$, ein spezifischer Antikörper für das Cytokeratin 8, hinterlegt bei der ECACC unter der Hinterlegungsnummer 87110601 (siehe nachfolgende Tabelle 4); $K_s18-27IV$, ein spezifischer Antikörper für das Cytokeratin 18,

2

hinterlegt bei der ECACC unter der Hinterlegungsnummer 8711301 (siehe nachfolgende Tabelle 5); $K_s$ 18-9B1, ein spezifischer Antikörper für das Cytokeratin 18, hinterlegt bei der ECACC wie in Tabelle 6 angegeben und VIM 3B4, ein für Vimentin spezifischer Antikörper, der bei der ECACC hinterlegt ist unter der Hinterlegungsnummer 87110602 (siehe nachfolgende Tabelle 7).

Vorzugsweise werden durch kombinierte Anwendung der vier an sich bekannten Testverfahren Immunblot ("Western-Blot"), Dot-Blot, ELISA und Immunfluoreszenzmikroskopie geeignete Antikörper ausgewählt.

Dabei wird vorzugsweise wie folgt verfahren:

I. Immunblot ("Western-Blot" nach Towbin et al., (1979), Proc. Natl. Acad. Sci., USA 76, 435).

IF-Proteinstandards, das sind die alpha-helikalen Mittelstücke, zeigen nach elektrophoretischer Auftrennung auf Polyacrylamidgelen (SDS-PAGE nach Lämmli, 1970, Nature 227,68) ein $M_r$ von ca. 38.000 bis 43.000. Nur solche Antikörper, die im Blot außer mit dem intakten Monomer des IF-Proteins auch mit dem Spaltstück positiv reagieren, werden ausgewählt. Für die Cytokeratine 8, 18 und 19 sind Immunblot-Reaktionen im Detail beschrieben in Franke et al., (1987), Exp.Cell Res., 173, 17-37, und in Hatzfeld et al. (1987) J. Mol. Biol. 197, 237-255.

II. Dot-Blot nach Franke et al. (1987), Exp.Cell Res. 173, 17-37.

Unter Pufferbedingungen (mit Zusatz von 4 M Harnstoff), welche die Bildung von Tetrameren und Protofilamenten begünstigen, werden die gereinigten IF-Proteine und nach bekannten Verfahren hergestellte alpha-helikale Mittelstücke im Vergleich mit Überstandsfraktionen auf Nitrozellulose aufgebracht und die Immunreaktion der Antikörper untersucht. Überstandsfraktionen werden gewonnen, indem eine Gewebeprobe homogenisiert wird, das Homogenat mit einer Protease (zum Beispiel Chymotrypsin) inkubiert wird, nach einer bestimmten Inkubationszeit die Enzymaktivität gestoppt beziehungsweise das Enzym abgetrennt und das Homogenat zentrifugiert wird. Im Überstand befinden sich durch proteolytische Fragmentierung in Lösung gegangene alpha-helikale Mittelstücke der IF-Proteine. Im Unterschied zum Immunblot-Verfahren ("Western-Blot") werden diese Fragment-Proteine beim Dot-Blot vor ihrer Immunreaktion nicht denaturiert, sondern reagieren mit dem jeweiligen Antikörper in einer ihrem nativen Zustand entsprechenden Anordnung der Polypeptid-Ketten.

III. ELISA (enzyme linked immunosorbent assay)

Dieses bekannte Verfahren wird hier nur für IF-Proteine verwendet, die unter physiologischen Bedingungen löslich sind. Das sind in diesem Fall die als Standards präparierten alpha-helikalen Mittelstücke, gelöst in Pufferlösung beziehungsweise Kontrollserum und anderen natürlichen Körperflüssigkeiten und in Überstandsfraktionen nach Gewebe-Homogenisierung und -Verdauung. Bei diesem Test werden die IF-Fragmentproteine nicht denaturiert, sondern reagieren mit dem jeweiligen Antikörper in ihrem nativen Zustand.

IV. Immunfluoreszenz-Mikroskopie

Von Geweben werden Gefrier-Dünnschnitte angefertigt, dabei werden die Zellen aufgebrochen und das Intermediärfilament-Netzwerk für Antikörper zugänglich gemacht. Die Immunreaktion findet daher am natürlichen Ort der Antigene, das sind hier die intakten Intermediärfilamentproteine, statt unter nicht denaturierenden Bedingungen.

Vorzugsweise werden nur solche Antikörper, die nach allen unter I bis IV genannten Verfahren eindeutig positiv bewertet werden, in Verbindung mit der Erfindung eingesetzt.

Der Test erfolgt gegenüber dem Standard, weil dieser reproduzierbare und vergleichbare Bedingungen schafft, wie sie für die Beurteilung der getesteten Antikörper vorteilhaft sind.

Einen Standard gewinnt man vorzugsweise dadurch, daß aus dem entsprechenden Gewebe durch Extraktion und Reinigung die einzelnen IF-Proteine gewonnen werden.

Sollen Cytokeratin-Mittelstücke hergestellt werden, dann werden äquimolare Mengen aus einem basischen Cytokeratin und einem dem gleichen Gewebetyp zugeordneten sauren Cytokeratin in Harnstoff gelöst, und durch Herausdialysieren des Harnstoffs bilden sich zunächst die Tetramere.

Soll ein IF-Mittelstück gebildet werden, das nicht aus Cytokeratinen stammt, dann können aus dem gereinigten Protein gleichen Typs unmittelbar die Tetramere als Vorstufe zu Protofilamenten und Filamenten durch Rekonstitution gebildet werden.

In beiden Fällen werden dann durch kontrollierte Verdauung mit Protease, vorzugsweise Chymotrypsin, die alpha-helikalen Mittelstücke als Tetramere freigesetzt, dann isoliert und gereinigt und können dann als Standard eingesetzt werden.

Die bei der Anwendung dieser Erfindung als Standards verwendeten alpha-helikalen Mittelstücke können durch Isolierung aus beliebigen Zell-Linien oder Geweben gewonnen werden, die IF-Proteine produzieren.

Die Rekonstitution der gereinigten Einzel-IF-Proteine kann beispielsweise unter Einsatz spezifisch gereinigter, nichtkeratinöser IF-Proteine oder durch Kombination äquimolarer Mengen der Cytokeratine des sauren und des basischen Typs erfolgen, mit anschließendem Dialysieren der Tetramere gegen niedrigkonzentrierte Salzpuffer. Der Aufbau in Protofilamente und IFs kann zum Beispiel durch eine elektronenmikroskopische Analyse negativ gefärbter Proben nach Hatzfeld, M., et al., J. Cell Biol. 101:1826-1841 (1985), kontrolliert werden.

Die rekonstituierten IF-Proteine werden danach einer limitierten Proteolyse unterworfen, vorzugsweise unter Einsatz von Trypsin, Chymotrypsin oder Thrombin. Das Ausmaß des proteolytischen Abbaus kann durch Gel-Elektrophorese zur Maximierung des Anteils an Polypeptidfragmenten im Bereich von $M_r$ 38.000 bis 40.000 kontrolliert werden. Dann werden die alpha-helikalen Mittelstücke isoliert, vorzugsweise durch Gel-Chromatografie oder eine HPLC mit Phasenumkehr.

Bei der Solubilisierung der zu identifizierenden oder der rekonstituierten IF-Proteine werden zunächst durch den proteolytischen Abbau die an den alpha-helikalen Mittelstücken hängenden Kopf- und Schwanzstücke abgeschnitten. Bei weiterer Einwirkung des proteolytischen Abbaus kann es zu einer einfachen Teilung etwa in der Mitte der alpha-helikalen Mittelstücke kommen, da diese dort einen kurzen Abschnitt aufweisen, auf dem die alpha-helikale Struktur unterbrochen ist. Es fallen auf diese Weise durch einfache Teilung zwei Mittelstückfragmente von jedem Mittelstück an, von denen mindestens eines ein dem betreffenden Antikörper zugeordnetes Epitop trägt.

Für das Identifizierungsverfahren nach der Erfindung und die Standardisierung ist es nicht nachteilig und verfälschend, wenn einige oder alle der alpha-helikalen Mittelstücke in dieser Weise einfach geteilt werden. Die Gesamtzahl der für die Antikörper zur Verfügung stehenden Epitope bleibt davon unberührt und damit auch die quantitativen Ergebnisse des Tests.

Man gewinnt auf diese Weise einen gewissen Toleranzspielraum bei der Solubilisierung, indem man diese so anlegt, daß mit Toleranzzugabe alle Mittelstücke isoliert und damit löslich sind, und hinnimmt, daß durch die Toleranzzugabe einige oder alle Mittelstücke einfach geteilt sind.

Es werden deshalb vorzugsweise solche Antikörper eingesetzt, deren Epitope durch die einfache Teilung unbeeinflußt an jeweils einem dieser Mittelstückfragmente sitzen. Zur Quantifizierung der Identifikation genügt es, für die geteilten Mittelstücke die die Epitope aufweisenden Mittelstückfragmente mitzuzählen.

Als Antikörper werden vorzugsweise eingesetzt:

$K_s$ 8-17.2 zur Identifizierung eines alpha-helikalen Mittelstücks, von dem mindestens ein Monomer aus dem Cytokeratin No. 8 stammt,

$K_s$ 18-27 IV und/oder $K_s$ 18-9B1 zur Identifizierung eines alpha-helikalen Mittelstücks, von dem mindestens ein Monomer aus dem Cytokeratin No. 18 stammt,

$K_s$ 19.2 zur Identifizierung eines alpha-helikalen Mittelstücks, von dem mindestens ein Monomer aus dem Cytokeratin No. 19 stammt,

$K_s$ pan 1-8 zur Identifizierung eines alpha-helikalen Mittelstückes, von dem mindestens ein oder mehrere Monomere aus einem oder mehreren der Cytokeratine No. 1 bis 8 stammen, und/oder

VIM 3B4 zur Identifizierung eines alpha-helikalen Mittelstücks des Vimentins.

Diese Antikörper haben sich als eindeutig identifizierend erwiesen, wie weiter unten noch anhand der Beispiele näher dargelegt wird. Die genannten Antikörper werden nachfolgend beschrieben:

TABELLE 2

Monoklonaler Antikörper gegen Cytokeratine der basischen
Cytokeratin-Unterfamilie (Nr. 1-8)

Antikörper: Der AntikörperK$_S$pan 1-8 wird
produziert von der Hybridom-
Zellinie K$_S$ pan 1-8.
Die Hybridom-Zellinie K$_S$ pan
1-8 ist hinterlegt am 19. Jan.
1988 unter ECACC 88011901.

Kategorie: Monoklonaler Antikörper aus
der Maus: Stamm BALB/c und
Fusionspartner: Myeloma-Zell-
linie X63-Ag8.653

Immunoglobulinklasse: IgG2a

Antigen: Cytoskelettproteine aus kultivierten menschlichen
MCF-7-Zellen.

Reagierendes Polypeptid: Das den verschiedenen Cytokeratinen der basischen
(Typ II) Unterfamilie
(CK 1-8) gemeinsame Epitop

Reaktive Arten: Mensch, Rind

Erkannte Strukturen,
Antigene und Gewebe: Alle Epithele einschl. beispielsweise Epidermis, Zahnfleisch, Zunge, Ösophagus,
Hepatozyten und Gallengänge,

|  | Dünndarm, Kolon, Vagina- und Cervixschleimhaut, Brustdrüsenepithel und Myoepithelien, retikuläres Epithel des Thymus, haarbildende Zellen und Haar. |
|---|---|
| Positive Reaktivität in kultivierten Zell-Linien: | MCF-7, RT-112, Detroit 562, RPMI 2650, HT-29, SCC-12 (alle menschlich); BMGE, MDBK (alle Rind) |
| Erkannte menschliche Tumore: | Alle bisher untersuchten Karzinome einschl. Basaliom, Stachelzellenkarzinom der Epidermis, Plattenepithelkarzinom von Zunge, Ösophagus, Lunge und Cervix, Adenokarzinom von Lunge, Kolon und Cervix, Nierenzellenkarzinom, Mammakarzinom, Leberzellenkarzinom. |
| Anwendung: | Pathologie (insb. Differentialdiagnose von Tumoren, auch zwischen verschiedenen Karzinomarten), Zelltypisierung in Produktion, Forschung. |

TABELLE 3

Monoklonaler Antikörper gegen Cytokeratin 19

| Antikörper: | Der Antikörper $K_S$ 19.2 wird produziert von der Hybridom-Zellinie $K_S$ 19.2. Die Hybridom-Zellinie ist hinterlegt am 13. Nov. 1987 unter ECACC 87111302 |
|---|---|
| Kategorie: | Monoklonaler Antikörper aus der Maus: Stamm BALB/c und Fusionspartner: Myeloma-Zellinie NSO |
| Immunoglobulinklasse: | IgG2b |
| Antigen: | Cytoskelett aus kultivierten menschlichen MCF-7-Zellen |
| Reagierendes Polypeptid: | Ausschließlich Cytokeratin 19 |

6

$(M_r \text{ ungefähr} = 43.000)$

Reaktive Arten:       Mensch, Rind

Erkannte Strukturen,
Antigene und Gewebe:      Nur mit bestimmten Epithelien reaktionsfähig. Positive Epithelien sind: Intestinalschleimhaut, Gallengänge, Pankreas, Magenschleimhaut, Sammelwege von Niere, Ureter, Blasenurothel, Samenblase, Prostata, Mesothel, Eileiter, endometrisches Epithel, Endocervix, Bronchialepithel, Brustdrüse (Gangsystem, Acini) retikuläres Epithel des Thymus, Larynx- und Pharynx-Epithelien, Drüsenepithelien der Haut, Basal-Zellschichten einer Reihe geschichteter Epithelien (zum Beispiel Analepidermis, Vagina, Exocervix, Urethra, Ösophagus, Zungen- und Mundschleimhaut, Zahnfleisch). Negative Epithelien sind zum Beispiel: Epidermis der meisten Körperregionen, suprabasale Schichten in den meisten Stadien verschiedener mehrschichtiger Epithelien (zum Beispiel Exocervix, Vagina, Zunge, Ösophagus), Hepatozyten, azinöse Zellen des Pankreas, proximale Nierentubuli, Hoden, alle Mesenchym-Gewebe, die meisten Muskelgewebsarten, Neuralgewebe, Linsengewebe, Endothelien und andere Gefäßkomponenten.

Positive Reaktivität
in kultivierten menschlichen Zell-Linien:      MCF-7, HT-29, HeLa, RT-112, Detroit 562, RPMI 2650, SSC-12

Erkannte menschliche
Tumore:      Adenokarzinome von Kolon, Magen, Pankreas, Gallenblase, Endometrium, Cervix, Cholangiokarzinom der Leber, Nierenzellenkarzinom, Carcinoma transitiocellulare der Blase, Ovarialkarzinome, Platten-

epithelkarzinom der Cervix, Mesotheliom, Plattenepithel-karzinom von Bronchien und Lunge, großzelliges Karzinom der Lunge, kleinzelliges Karzinom der Lunge (intermediärer Typ), karzinoider Tumor der Bronchien, Brustkarzinom.

Anwendung:      siehe Tabelle 2

Referenzliteratur:

1. Franke, W.W., et al., Cell Typing of Epithelia and Carcinomas of the Female Genital Tract Using Cytoskeletal Proteins as Markers. Banbury Report 21:Viral Etiology of Cervical Cancer. Cold Spring Harbor Laboratory; pp. 121 (1986)

2. Moll, R., et al., Cell 31:11-24 (1982)

TABELLE 4

Monoklonaler Antikörper gegen Cytokeratin 8

| | |
|---|---|
| Antikörper: | Der Antikörper $K_S$ 8-17.2 wird produziert von der Hybridom-Zellinie $K_S$ 8-17.2. Die Hybridom-Zellinie ist hinterlegt am 6. Nov. 1987 unter ECACC 87110601 |
| Kategorie: | Monoklonaler Antikörper aus der Maus: Stamm BALB/c und Fusionspartner: Myeloma-Zell-linie X63-Ag8.653 |
| Immunglobulinklasse: | IgG 1 |
| Antigen: | Cytoskelett aus MCF-7 Kultur-zellen (Human-Zellinie) |
| Reagierendes Polypeptid: | Ausschließlich Cytokeratin 8 ($M_r$ ungefähr = 52.500) |
| Reaktive Arten: | Mensch, Rind |
| Erkannte Strukturen, Antigene und Gewebe: | Positive Epithele schließen ein: Leber (Hepatozyten, Gallengänge), Darm, Drüsen-epithel der Zunge und alle einfachen Epithelien |

Positive Reaktionen mit
menschlichen Kulturzellen:  MCF-7, HeLa, A-431

Erkannte menschliche
Tumoren:  Positive Karzinome schließen
ein: Hepatozelluläres Karzinom,
Adenokarzinom von Colon, Lunge,
Brust.

Anwendung:  wie in Tabelle 2.


TABELLE 5

Monoklonaler Antikörper gegen Cytokeratin 18

Antikörper:  Der AntikörperK$_S$ 18-27IV wird
produziert von Hybridom-
Zellinie K$_S$ 18-27IV.
Die Hybridom-Zellinie K$_S$
18-27IV ist hinterlegt am
13. Nov. 1987 unter
ECACC 87111301

Kategorie:  Monoklonaler Antikörper aus
der Maus: Stamm BALB/c und
Fusionspartner: Myeloma-Zell-
linie SP2/OAg14

Immunglobulinklasse:  IgG 1

Antigen:  Cytoskelett aus MCF-7 Kulturzellen

Reagierendes Polypeptid:  Ausschließlich Cytokeratin 18
($M_r$ ungefähr = 45.000)

Reaktive Arten:  Mensch, Rind

Erkannte Strukturen,
Antigene und Gewebe:  Positive Epithelien schließen
ein: Cytokeratinfilamente in
einschichtigen Epithelien und
Drüsen, zum Beispiel in Leber
(Hepatocyten, Gallengänge),
Darm, Blase (Urothel); in mehrschichtigen Epithelien (zum
Beispiel Vagina, Zunge) nur
bestimmte Zellen in der
Basalzellschicht.

Erkannte menschliche
Tumore:

Kolon-Karzinom, Brustkarzinom, Adenokarzinome verschiedener Organe.

Positive Reaktionen mit
menschlichen Kulturzellen:

MCF-7, HeLa, A-431, Detroit 562, RT-112

Anwendung:

wie in Tabelle 2.

TABELLE 6

Monoklonaler Antikörper gegen Cytokeratin 18

Antikörper:

Der Antikörper $K_S$ 18-9B1 wird produziert von der Hybridom-Zellinie $K_S$ 18-9B1. Die Hybridom-Zellinie $K_S$ 18-9B1 ist hinterlegt am 05. Jan. 1989 unter ECACC 89010505

Kategorie:

Monoklonaler Antikörper aus der Maus: Stamm BALB/c und Fusions-partner: Myeloma-Zellinie X63-Ag8.653

Immunglobulinklasse:

IgG1

Antigen:

Rekonstituierte Cytokeratine 8:18 aus Rind (Kalbsleber)

Reagierendes Polypeptid:

Cytokeratin 18
($M_r$ ungefähr = 45.000)

Reaktive Arten:

Mensch, Rind

Erkannte Strukturen,
Antigene und Gewebe:

Cytokeratinfilamente in ein-schichtigen Epithelien und Drüsen, z.B. in Leber (Hepato-cyten, Gallengänge), Darm (Epithel), Blase (Urothel) und in mehrschichtigen Epithelien (z.B. Vagina, Zunge) nur be-stimmte Zellen in der Basal-zellschicht.

| Erkannte meschliche Tumore: | Coloncarcinome, Mammacarcinome und deren Lymphknoten-Metastasen, Adenocarcinome verschiedener Organe |
|---|---|
| Positive Reaktionen mit menschlichen Kulturzellen: | MCF-7, HeLa, A-431, Detroit 562, RT-112 |
| Anwendung: | wie in Tabelle 2. |

TABELLE 7

Monoklonaler Antikörper gegen Vimentin

| Antikörper: | Der Antikörper VIM 3B4 wird produziert von der Hybridom-Zellinie VIM 3B4. Die Hybridom-Zellinie VIM 3B4 ist hinterlegt am 6. Nov. 1987 unter ECACC 87110602 |
|---|---|
| Kategorie: | Monoklonaler Antikörper aus der Maus: Stamm BALB/c und Fusionspartner: Myeloma-Zell-linie X63-Ag8.653 |
| Immunglobulinklasse: | IgG 2a |
| Antigen: | Aus Rinderlinsen gereinigtes Vimentin |
| Reagierendes Polypeptid: | Spezifisch für Vimentin ($M_r$ ungefähr = 57.000 auf SDS-PAGE) |
| Erkannte Arten (bis jetzt untersucht): | Mensch, Rind, Nagetiere (Ratte, Maus, Hamster), Huhn, Amphibien (Xenopus) |
| Gewebespezifität: | Alle Zellen, die mit Antiseren gegen Vimentin positiv waren, reagieren auch positiv mit dem Antikörper: Zellen mesenchyma-len Ursprungs, auch Endothel-zellen und bestimmte Zellen glatter Gefäßmuskulatur, Fibro-blasten, Bindegewebe, alle Arten von Blutzellen, ein-schließlich Thymozyten, inter- |

stitielle und Sertoli-Zellen des Hodens, Follikelzellen des Ovars; Koexpression von Vimentin mit anderen Intermediärfilamenten wird erkannt.

Positive Reaktionen mit getesteten Kulturzell-Linien:

RD Zellen, Glioma-Zellen, Fibroblasten (SV-80, 3T3), BHK

Erkannte menschliche Tumore:

Alle Vimentin exprimierenden Tumore, wie zum Beispiel Sarkome (einschließlich Myosarkome), Lymphome, Melanome etc.

Anwendung:  wie in Tabelle 2.

Zur Identifizierung der Neurofilamente und Gliafilamentproteine können Antiseren gegen Neurofilamentproteine (NF-8, im Handel beziehbar von Progen, Heidelberg, BRD) sowie Antikörper gegen Gliafilamentproteine (Meerschweinchen-Antiserum GF-100, Hybridom-Zell-Linie GF 12.24, im Handel beziehbar von Progen) verwendet werden. Ein weiterer Antikörper gegen Neurofilamentproteine ist von Boehringer Mannheim, BRD im Handel.

Ein bevorzugtes Verfahren ist dadurch gekennzeichnet, daß alpha-helikale Mittelstücke in Körperflüssigkeiten, zum Beispiel Serum, immunologisch identifiziert werden durch Anwendung eines ELISA-Tests, daß dafür als an einer Titerplatte zu fixierende Fänger-Antikörper solche Antikörper nach Anspruch 1 eingesetzt werden, die mehrdeutig immunologisch reagieren, daß als Detektor-Antikörper solche Antikörper nach Anspruch 1 eingesetzt werden, die eindeutig immunologisch reagieren, und daß die mit der zu untersuchenden Lösung gewonnenen Titerergebnisse verglichen werden mit Titerergebnissen, die mit einer eingestellten Lösung eines Standards gewonnen wurden, welcher Standard nach Anspruch 2 oder 3 aus dem der zu untersuchenden Lösung entsprechenden Gewebetyp gewonnen wurde.

Durch die Anwendung eines mehrdeutigen Fänger-Antikörpers kann man ein und dieselbe Titerplatte für die Identifizierung verschiedener IF-Proteine einsetzen, indem eine eindeutige Identifizierung durch die Anwendung der eindeutigen Detektor-Antikörper erfolgt.

In diesem Sinne empfiehlt sich für Cytokeratine der $K_s$ pan 1-8 als Fänger-Antikörper, weil dieser auf mindestens eines der in jedem Cytokeratin-Filament enthaltenen basischen Monomere der Cytokeratine 1 bis 8 gerichtet ist, so daß ausnahmslos alle Cytokeratine eingefangen werden, und dann aufgrund der damit gepaarten sauren Monomere 9 bis 19 von den spezifischen Detektor-Antikörpern identifiziert werden können, indem als Detektor-Antikörper für eines der Cytokeratine 1 bis 19 zum Beispiel die in den Tabellen 3 bis 6 beschriebenen Antikörper gegen Cytokeratin 8, 18 und 19 eingesetzt werden.

Die Detektor-Antikörper können durch Radioaktiva, Enzyme oder Fluoreszenzfarbstoffe markiert sein.

Zur Identifizierung von Antikörpern gegen IF-Proteine in einer Probe kann ein "Festphasentest" durchgeführt werden. Das Antigen, das heißt in diesem Fall das entsprechende IF-Protein, wird immobilisiert, mit der Probe inkubiert, gewaschen und der aus der Probe gebundene Antikörper identifiziert, zum Beispiel mit einem zweiten, markierten Antikörper.

Je nach der Gewebsprobe und der Art ihrer Vorbehandlung und der Durchführung des Tests wird man mehr oder weniger der tatsächlich vorhandenen IF-Proteine über ihre Mittelstücke identifizieren. Deshalb gibt unter Umständen die absolute Menge der gefundenen fremden, also nicht nativ zu dem untersuchten Gewebe gehörigen Mittelstücke keine erschöpfende Aussage. Eine bessere Aussage kann man dagegen erwarten, wenn man die gefundenen fremden IF-Proteine zu den für das betreffende Gewebe typischen, vorhandenen IF-Proteinen ins Verhältnis setzt. Das geschieht vorzugsweise dadurch, daß die in der Gewebeportion vorhandenen Intermediärfilamentproteine nach ihrem Ursprungsgewebe bestimmt und quantifiziert ermittelt werden, daß die gefundenen Mengen der der untersuchten Gewebeportion ursprungsmäßig nicht zugeordneten Intermediärfilamentproteine zu der für die dem untersuchten Gewebe ursprungsmäßig zugeordneten Intermediärfilamentproteine gefundenen Menge ins Verhältnis gesetzt wird und daß dieses Verhältnis an einem nicht pathologischen Standard-Verhältnis gemessen wird.

Krebsherde sondern bestimmte Zellen ab, die sich bevorzugt in den nächstgelegenen Lymphknoten sammeln können (sogenannte Lymphknotenmetastasen). Diesen Umstand macht sich eine Weiterbildung der Erfindung zunutze, die dadurch gekennzeichnet ist, daß zur Aufdeckung von kanzerogenen Herden eine Lymphgewebeportion untersucht wird und die Menge des gefundenen Vimentinproteins zur Menge der gefundenen, jeweils anderen Gewebetypen zugeordneten Intermediärfilamentproteine ins Verhältnis gesetzt wird.

Die nachstehenden Beispiele dienen der Veranschaulichung der Verfahren der vorliegenden Erfindung.

BEISPIEL 1

Verfahren zur Gewinnung eines Standards

Die angewendeten Methoden werden am Beispiel der Cytokeratine beschrieben; die Art der Aufarbeitung kann jedoch auf alle IF-Proteine übertragen werden.

1.1. Reinigung der intakten Polypeptide

Humancytokeratine (zum Beispiel 8, 18, und 19) wurden im wesentlichen wie bei Achtstaetter, T., et al., Methods Enzymol. 134:355-371 (1986), beschrieben, aus der Humankulturzellinie MCF-7 gewonnen. Rindercytokeratine 8, 18 und 19 (zur Nomenklatur vergleiche Bader, B.L., et al., EMBO J. 5:1865-1875 (1986)), wurden aus Urothelzellen der Blase durch Abschaben der inneren Oberfläche von Rinderblasen gewonnen. Die abgeschabte Zellschicht wird homogenisiert (im wesentlichen beschrieben bei Achtstaetter T., et al., supra). Einzelne Cytokeratin-Polypeptide wurden mittels Anionen-Austauscher-Chromatografie auf DEAE-Zellulose (DE 52; Whatman Chemical Separation Inc., Clifton, NJ, USA) in einem 8 M Harnstoff-Puffer (8 M Harnstoff, 2,5 mM Dithioerythritol, 30 mM Tris-HCl (pH 8,0)) chromatografisch gereinigt, im wesentlichen wie beschrieben bei Hatzfeld & Franke, 1985; Achtstaetter et al., 1986; Bader et al., 1986; Quinlan et al., 1986. Kurz beschrieben, wurde Cytoskelettmaterial für 2 Stunden in 9,5 M Harnstoff (5 mM Dithioerythritol, 10 mM Tris-HCl (pH 8,0)) extrahiert und der nach Zentrifugation bei 100.000 x g ( g = Gravitationskonstante) erhaltene Überstandsextrakt wurde gegen einen 8 M Harnstoff-Puffer dialysiert und auf eine DEAE-Zellulosesäule, die mit diesem Puffer equilibriert wurde, aufgebracht. Gebundenes Protein wurde mit einem 0 bis 100 mM Guanidinium-HCl-Gradienten eluiert. Die Polypeptid-Zusammensetzung wurde durch eine SDS/Polyacrylamid-Gel-Elektrophorese (PAGE) kontrolliert. Die vereinigten Fraktionen wurden einer weiteren Reinigung durch eine Hochdruck-Flüssig-Chromatografie mit Phasenumkehr unterworfen, wobei 0,01% (v/v) Trifluor-Essigsäure (TFA) (Fluka, Buchs, Schweiz) als wässriges Lösungsmittel A, 0,07% (v/v) TFA in Azetonitril (chromatografische Qualität, Merck Darmstadt, BRD) als organische Phase (Lösungsmittel B) und eine BioRad-RP-304-Säule mit Phasenumkehr (BioRad Laboratories, Richmond, CA, USA) verwendet wurden. Die Peakfraktionen wurden gesammelt, das Azetonitril durch Vakuumverdampfung entfernt und die Fraktionen gefriergetrocknet.

1.2. Rekonstituierung der gereinigten Polypeptide zu Protofilamenten und IF-Proteinen

Die gereinigten Cytokeratine wurden in Puffer, der 9,5 M Harnstoff enthält, gelöst. Äquimolare Mengen an Typ I und Typ II Cytokeratin wurden bei einer Endkonzentration von etwa 0,5 mg/ml gemischt, und die Protofilamente und IF-Proteine wurden erhalten, indem die Polypeptidlösung gegen Puffer niedriger Ionenstärke dialysiert wurde. (Es wurden die Puffer, die bei Hatzfeld, M. und Franke, W.W., J. Cell. Biol. 101:1826-1841 (1985) beschrieben sind, verwendet.) Die Bildung von Protofilamenten und IF-Proteinen wurde elektronenmikroskopisch durch Negativ-Kontrastierung der Probe kontrolliert (vergleiche Hatzfeld M. und Franke, W.W., supra).

1.3. Darstellung alpha-helikaler Cytokeratin-Mittelstücke durch limitierte Proteolyse

Der proteolytische Abbau wurde mit verschiedenen Proteasen durchgeführt. In einer typischen Präparation wurden Chymotrypsin (EC 3.4.21.1 aus Rinderpankreas, zum Beispiel von der Fa. Sigma, München) in einem Enzym-zu-Substrat-Verhältnis (Gewicht/Gewicht) von 6,6:1000 für das Cytokeratin 8:18 Paar und im Verhältnis 9:1000 für das Cytokeratin 8:19 Paar eingesetzt. Für jede Chymotrypsin-Charge mußte die Abdauzeit optimiert werden. Der proteolytische Abbau wurde durch gelelektrophoretische Analysen der Abbauprodukte kontrolliert und für den maximalen Anteil an stabförmigen Mittelstücken ($M_r$ = 38.000 - 40.000) optimiert. Das Optimum liegt bei zirka 20 min bei 30°C. Nach der entsprechenden Abdauzeit wurde die Enzymaktivität durch Zugabe von 5 mM Phenylmethylsulfonylfluorid gestoppt.

1.4. Reinigung der alpha-helikalen Mittelstücke

Die proteolytischen Mittelstücke und deren einfache Fragmente wurden entweder chromatografisch auf einer Sepharose CL6B Säule oder direkt über Umkehrphasen-Hochleistungsflüssigkeitschromatografie getrennt, und zwar auf einer BioRad RP304-Säule mit Phasenumkehr unter Verwendung des im obigen Abschnitt 1 beschriebenen Lösungsmittelsystems. Zur weiteren Reinigung wurden die Hauptfraktionen mit Lösungsmittel A zur Reduzierung der Azetonitril-Konzentration auf ca. 20% (v/v) verdünnt und dann direkt auf eine My-Bondapak C18-Säule mit Phasenumkehr (Waters Associates, Milford, MA) aufgebracht. Alle Hauptfraktionen wurden lyophilisiert und die Proben mittels 1- und 2-dimensionaler Gelelektrophorese auf Anwesenheit der alpha-helikalen Mittelstücke untersucht. Die gereinigten alpha-helikalen Mittelstücke, die zum Teil einfach geteilt sind zu jeweils zwei Mittelstückfragmenten, da die Schnittstelle an einem kurzen mittleren Abschnitt, an dem die helikale Struktur unterbrochen ist, liegt, wurden als Referenzmaterial beziehungsweise Standard-Material für die Eichung des Nachweissystems und für Immunisierungen eingesetzt.

BEISPIEL 2

Auswahl und Herstellung geeigneter Antikörper

Spezifische Antikörper gegen Intermediärfilamentproteine, sowohl eigene Entwicklungen als auch kommerziell erhältliche, wurden auf ihre Immunreaktivität mit den alphahelikalen Mittelstückfragmenten, wie sie als Standard-Material nach BEISPIEL 1 gewonnen wurden, untersucht mit folgenden Methoden:

2.1. Immunblot (Western-Blot; Reaktion mit denaturiertem Antigen)

Gereinigte Fragmentproteine (z.B. Cytokeratin 8:18-, Cytokeratin 8:19- oder Vimentin-Fragmente) und Cytoskelett-Präparationen aus Gewebeproben (z.B. Lymphknoten oder Leber) vor und nach proteolytischer Verdaureaktion mit Chymotrypsin (vergleiche Beispiel 3) wurden gelelektrophoretisch (Natriumdodecylsulfat-Polyacrylamid-Gelelektrophorese) aufgetrennt, die Proteine elektrophoretisch auf Nitrozellulose transferiert und mit den in Frage kommenden spezifischen Antikörpern inkubiert. Die Immunreaktion wurde über markiertes Protein A oder markierte Anti-Maus-Antikörper nachgewiesen. Antikörper, die mit den alpha-helikalen Mittelstücken (Mr 38000-40000; Mr 20000-22000 im Falle der basischen Keratine) eine Immunreaktion zeigten, wurden ausgewählt.

2.2. Dot-Blot (Reaktion mit nativem bzw. renaturiertem Antigen)

Ca. $2 \times 10^{-6}$ g gereinigte IF-Proteine (gelöst in $50 \times 10^{-6}$ 1 50mM $Na_2HPO_4$-Puffer, pH 7,4) bzw. Überstandsfraktionen von homogenisierten Gewebeproben nach Chymotrypsinverdau werden direkt an Nitrozellulose gebunden (z.B. in einer SRC 96 Minifold I Dot-Blot-Apparatur von Schleicher u. Schuell, Dassel, BRD) und mit den in Frage kommenden, spezifischen Antikörpern inkubiert. Der weitere Arbeitsgang ist wie unter 1. beschrieben.

2.3. ELISA (Reaktion mit nativem bzw. renaturiertem Antigen)

$500 \times 10^{-9}$g gereinigte Fragmentproteine (gelöst in $100 \times 10^{-6}$1 50 mM $NaHCO_3$-Puffer, pH 9,6) bzw. 2 $\times 10^{-6}$g (in $100 \times 10^{-6}$1) Protein der Überstandsfraktionen von homogenisierten Gewebeproben nach Chymotrypsinverdau werden pro Vertiefung in einer 96-Loch Mikrotiterplatte inkubiert und gebundenes Protein mit den in Frage kommenden spezifischen Antikörpern inkubiert. Der weitere Arbeitsgang ist wie unter 1. beschrieben.

2.4. Immunfluoreszenz-Mikroskopie. Standardmethode, eingehend beschrieben z.B. bei Ciocca D.R. und Bjercke R.J. (1986) in Methods Enzymol. 121, 562-579.

Antikörper, die nach den o.g. Methoden positiv waren, sind $K_s$ 19.2; $K_s$18-9B1; $K_s$ 18-27 IV; $K_s$ 8-17.2; $K_s$ pan 1-8; VIM 3B4.

2.5. Herstellung monoklonaler, gegen alpha-helikale Mittelstücke gerichtete Antikörper

Zur Herstellung spezifischer Antikörper wurden nur in vitro rekonstituierte Filamente, die aus den jeweiligen gereinigten Polypeptiden gebildet wurden, zur Immunisierung injiziert. Um monoklonale Antikörper herzustellen, wurden weibliche, 6-8 Wochen alte BALB/c Mäuse immunisiert, indem ihnen Cytoskelett-Präparationen oder rekonstituierte Filamente mit 30-300 x $10^{-6}$ g Protein pro Injektion injiziert wurden. Die Antigene wurden in Phosphat-gepufferter Kochsalzlösung (PBS) suspendiert und für die erste Injektion mit Freund'schem Adjuvans (komplett) emulgiert. Bei allen folgenden Injektionen wurde Freund'sches Adjuvans (inkomplett) verwendet. Die Tiere wurden dreimal im Abstand von etwa drei Wochen subcutan gespritzt, und sie erhielten drei Tage vor der Zellfusion eine intraperitoneale Wiederholungs-Injektion mit 30-80 x $10^{-6}$ g Antigen. Die Milzzellen immunisierter Mäuse wurden mit Maus-Myelomzellen der Linie Sp2/0Ag14, X63-Ag8.653 und NSO/U (beschrieben wir bei Shulmann, M., et al., Nature 276:269-270 (1978); Kearney, I.F., et al., J. Immunol. 123:1548-1550 (1979); Clark and Milstein, Somatic Cell Genetics 7:657-666 (1981)) im Verhältnis 10:1 fusioniert, im wesentlichen wie es bei Koehler G. und Milstein C., Nature 256:495-497 (1975) beschrieben wurde. Die Hybridomüberstände wurden immunfluoreszenzmikroskopisch auf Gefrierschnitten von Human- und Rindergewebe (im wesentlichen beschrieben bei Achtstaetter T., et al., Differentiation 31:206-227 (1986)) oder auf Kulturzellen, die auf speziell beschichteten Objektträgern oder Deckgläschen gezüchtet wurden, getestet oder mit der Enzym-gekoppelten Immunadsorptionstechnik (ELISA), wobei die gereinigten Antigene zum Beschichten von Mikrotiterplatten eingesetzt wurden. Positive Klone wurden zweimal durch kontrollierte Ausverdünnung subkloniert. Ig-Subklassen wurden nach Ouchterlony, O. und Nilsson, L.A. (1978, in: Handbook of Experimental Immunology; Weir, D.M., ed., vol. 1, chapter 19, Blackwell Scientific Publications, Oxford, pp. 1-19) bestimmt.

2.6. Koppeln der Detektor-Antikörper an Peroxidase.

Die als Detektor-Antikörper bezeichneten monoklonalen Antikörper wurden nach einem von B. Tijssen (Laboratory techniques in biochemistry and molecular biology, vol. 15: Practice and theory of enzyme immunoassays, R.H. Burdon and P.H. van Knippenberg, eds., Elsevier Amsterdam, New York, Oxford; S. 238) beschriebenen Verfahren an Peroxidase gekoppelt:

5 mg Peroxidase werden in 0,5 ml Natriumcarbonatpuffer 100 mM; pH 9,2) gelöst und zum Koppeln vorbereitet, indem das Enzym 2 Stunden bei Raumtemperatur und unter Lichtabschluß mit 0,5 ml einer 10 mM NaIO$_4$-Lösung oxidiert wird. Danach wird der gewünschte Antikörper (10 mg gelöst in 2 ml 100 mM Natriumcarbonatpuffer, pH 9,2) zugesetzt, 0,5 g trockenes Sephadex® G-25 (Fa. Pharmacia, Freiburg) zugegeben und weitere 3 Stunden unter Lichtabschluß inkubiert. Das dabei entstandene Konjugat wird aus dem Sephadex-Material mit dem Natriumcarbonatpuffer eluiert und mit 1/20 Volumteil einer 0,5 % NaBH$_4$-Lösung in 0,1 mM NaOH gemischt. 30 min. später wird 1/10 Volumteil der gleichen Lösung zugesetzt und 1 Stunde bei 4°C inkubiert. Das Konjugat wird unter Vakuumdialyse gegen PBS dialysiert und auf ca. 0,5 ml konzentriert und anschließend auf einer Sephadex® G-200 (Fa. Pharmacia) Säule (1,0 x 50 cm) fraktioniert. Die Fraktionen (ca. 0,5 ml Volumen) werden auf ihren Anteil an Enzymaktivität und Antikörper getestet und die Fraktionen, die gleichzeitig hohe Ig-Konzentration und hohe Enzymaktivität aufweisen, zusammengefaßt.

BEISPIEL 3

Nachweis und Bestimmung von Metastasen in Lymphgewebe

3.1. Solubilisierung von IF-Proteinen, vorzugsweise deren alpha-helikale Mittelstücke

Es wird zunächst das Feuchtgewicht des Lymphknotengewebes ermittelt. Das Gewebe wird im dreifachen Volumen - bezogen auf das Feuchtgewicht - einer phosphatgepufferten Kochsalzlösung (PBS) (10 mM Natriumphosphat pH 7,4, 150 mM Natriumchlorid) mit Hilfe eines Messerhomogenisators bis zur breiartigen Konsistenz zerkleinert (es wird der Einsatz eines Polytron-Homogenisators der Firma Kinematica, Luzern/Schweiz empfohlen). Das Homogenat wird mit Chymotrypsin inkubiert.

Zu diesem Zweck wird Chymotrypsin verwendet, das zuvor an eine Matrix (CNBr-aktivierte Sepharose® 4B, Fa. Pharmacia, Freiburg) gebunden worden ist:1 g CNBr-aktivierte Sepharose® 4B werden 15 min in lmM HCl gequollen (Gelvolumen ca. 3,5 ml/g) und mit insgesamt 200 ml lmM HCl gewaschen. Die Salzsäurelösung wird abgesaugt und das Matrixmaterial mit 5 ml Kopplungspuffer (0,5 M NaCl, 0,1 M NaHCO$_3$, pH 8,0) gewaschen. 10 mg Chymotrypsin werden in 5 ml Koppplungspuffer gelöst und mit dem Matrixmaterial in Kopplungspuffer 2 Stunden bei Raumtemperatur unter ständiger Bewegung inkubiert. Verbliebene, nicht abgesättigte Kopplungsstellen werden danach durch Zusatz von 5 ml 0,2 M Glycinlösung (pH 8,0) blockiert. Anschließend wird das Gelmaterial mit einem Überschuß an Kopplungspuffer (ca. 50 ml) und 10 ml Acetatpuffer (0,5 M NaCl, 0,1 M Natriumacetat, pH 4,0) gewaschen. Unter diesen Bedingungen werden ca. 60 % des eingesetzten Chymotrypsins gebunden, das heißt das Sepharose 4B-Gel enthält 1,7 mg/ml gekoppeltes Chymotrypsin. Zur besseren Handhabung wird das Gel zweifach in PBS verdünnt (Chymotrypsin-Konzentration 0,85 mg/ml).

Dem Gewebebrei wird Chymotrypsin (EC 3.4.21.1 aus Rinderpankreas, zum Beispiel von der Fa. Sigma, München) im Verhältnis 1:1000 (berechnet auf das Feuchtgewicht des Gewebes) zugesetzt. Es folgt ein Inkubationsschritt bei 30°C (vorzugsweise im Thermoblock, eventuell im Wasserbad). Durch Einstellen des Homogenats in Eis für 5 min wird die Abdaureaktion nach 30 min gestoppt. Das Homogenat wird 30 min bei $2 \times 10^4$ g zentrifugiert und der Zentrifugationsüberstand sofort abgenommen; das gekoppelte Chymotrypsin befindet sich im Sediment.

Unter diesen Bedingungen werden 80 bis 95 Gewichtsprozent des Materials der alpha-helikalen Mittelstücke in noch identifizierbarem Zustand aus den IF-Proteinen in die Überstandsfraktion freigesetzt und einige intakte IF-Proteine befinden sich ebenfalls in einem löslichen Stadium.

### 3.2. Ermittlung und Bestimmung des Vimentingehaltes

Im zentrifugierten Überstand wird Vimentin immunologisch durch einen Sandwich-ELISA bestimmt. Hierzu dient ein erstes Anti-Serum, GP-8, als Fänger-Antikörper, die gegen das alpha-helikale Mittelstück gerichtet sind, und ein zweiter monoklonaler Antikörper VIM-3B4 als Detektor-Antikörper, der gegen ein anderes Epitop des alpha-helikalen Mittelstückes, das von den ersten Epitopen unabhängig und verschieden ist, gerichtet ist. Der Fänger-Antikörper (GP8) wird in einer Konzentration von $10 \times 10^{-6}$ g/ml, gelöst in 50 mM $NaHCO_3$ (pH 9,6), auf Mikrotiterplatten beschichtet ($150 \times 10^{-6}$ l pro Vertiefung). Für die Standardkurve wird gereinigtes Vimentin-Fragment in Konzentrationen von 10 ng/ml bis 500 ng/ml (gelöst in Puffer: 150 mM NaCl, 10 mM $Na_2HPO_4$ pH 7,4, 0,05% Tween® 20) verwendet. Zur Messung der VimentinKonzentration im zentrifugierten Überstand wird dieser 1:100 bis 1:500 mit dem letztgenannten Puffer verdünnt. Der mit Peroxidase markierte Detektor-Antikörper VIM 3B4 wird mit Puffer (150 mM NaCl, 10 mM $Na_2HPO_4$ pH 7,4, 1% Rinderserumalbumin, 0,05% Tween® 20) auf eine Konzentration von $0,5 \times 10^{-6}$ g/ml verdünnt und mit $150 \times 10^{-6}$ l pro Vertiefung eingesetzt. Als Substrat wird o-Phenylendiamin oder ABTS (2,2'-Azino-di- (3-ethylbenzthiazolinsulfonat (6))) verwendet. Der so erhaltene Vimentinwert dient dann als Bezugsgröße für die quantitative Auswertung der weiteren Meßergebnisse.

### 3.3. Bestimmung der Cytokeratine und Ermittlung der Cytokeratingehalte

Im zentrifugierten Überstand werden die vorhandenen Cytokeratine immunologisch durch einen Sandwich-ELISA bestimmt. Hierzu dient ein erster monoklonaler Antikörper $K_s$ pan 1-8, der sogenannte Fänger-Antikörper, der gegen ein erstes, für die Cytokeratine 1 bis 8 typisches Epitop gerichtet ist. Der Fänger-Antikörper $K_s$ pan 1-8 wird in einer Konzentration von $20 \times 10^{-6}$ g/ml, gelöst in 50 mM $NaHCO_3$ (pH 9,6), auf Mikrotiterplatten beschichtet ($150 \times 10^{-6}$ l pro Vertiefung). Für die Standardkurve werden z.B. die gereinigten Cytokeratin-Fragmente in den Kombinationen Cytokeratin 8:18 und 8:19 in Konzentrationen von 5 ng/ml bis 500 ng/ml (gelöst in Puffer: 150 mM NaCl, 10 mM $Na_2HPO_4$ pH 7,4, 0,05% Tween 20) verwendet. Als Peroxidase-gekoppelte Detektor-Antikörper werden z.B. $K_s$ 18-27 IV und $K_s$18-9B1 (für Cytokeratin 18 Fragmente), $K_s$ 19.2 (für Cytokeratin 19 Fragmente) und $K_s$ 8-17.2 (für Cytokeratin 8 Fragmente) eingesetzt. Zur Messung der Cytokeratin-Konzentration im zentrifugierten Überstand wird dieser 1:100 mit Puffer (150 mM NaCl, 10 mM $Na_2HPO_4$, pH 7,4, 0,05 % Tween 20) verdünnt.

Zur Standardisierung werden bekannte Mengen des nach BEISPIEL 1 gewonnenen Cytokeratin-Standards dem Sandwich-ELISA unterworfen. Die Enzymaktivität, die der Konzentration eines jeden standardisierten Cytokeratins entspricht, wird gegen die Konzentration aufgetragen, um eine Standard-Kurve zu erhalten, von der die Konzentration unbekannter Mengen eines jeden Cytokeratins interpoliert werden kann.

Das Ausmaß von Karzinom-Metastasen kann ausgedrückt werden durch das Verhältnis des bestimmten Cytokeratins und des gemessenen Vimentins in der Gewebeprobe.

BEISPIEL 4

Quantitative Bestimmung von Cytokeratin 8:19 im Sandwich-ELISA auf Mikrotiterplatten

4.1. Beschichten der Mikrotiterplatten

In jede Vertiefung werden $2 \times 10^{-6}$ g Fänger-Antikörper $K_s$ pan 1-8 (gelöst in 100 - 150 $\times 10^{-6}$ l 50 mM Natriumcarbonatpuffer pH 9,6) pipettiert. Die Platte wird abgedeckt und über Nacht bei 4°C inkubiert.

4.2. Waschen und Blockieren

Die überschüssige Antikörperlösung aus jeder Vertiefung wird durch Absaugen entfernt. In jede Vertiefung werden 3 x hintereinander 200 $\times 10^{-6}$ l Waschpuffer (PBS-Tween: 150 mM NaCl, 10 mM Natriumphosphatpuffer pH 7,4, 0,05% Tween® 20) pipettiert und durch Umdrehen der Platte entfernt. Restfeuchtigkeit wird durch leichtes Ausklopfen der Platte auf mehrere Lagen Papierhandtücher entfernt. Jede Vertiefung wird mit 200 $\times 1.0^{-6}$ l. Abblockpuffer (150 mM NaCl, 10 mM Natriumphosphatpuffer pH 7,4, 0,05% Tween® 20, 1% Rinderserumalbumin, 5% Saccharose; bei längerer Aufbewahrungszeit werden außerdem 0,01% Thimerosal zugefügt) gefüllt und mindestens 1 Stunde bei Raumtemperatur inkubiert.

4.3. Inkubieren mit Antigen bzw. Serumproben

Standardprotein des Cytokeratin 8:19, gewonnen nach BEISPIEL 1 in Konzentrationen zwischen 5 ng/ml und 500 ng/ml (abhängig vom jeweiligen Detektor-Antikörper) wird in Kontrollserum (Monitrol von Merz & Dade oder Kontrollogen L und LU von Behring) aufgenommen. Das Kontrollserum wird ebenfalls in Abhängigkeit vom Detektor-Antikörper in Verdünnung von 1:10 bis 1:100 eingesetzt. Je Vertiefung werden 100 $\times 10^{-6}$ l Standardproteinlösung oder Serumproben (1:10 bis 1:100 verdünnt) pipettiert und 90 min. bei Raumtemperatur inkubiert. Danach wird 4 x mit 200 $\times 10^{-6}$ l Waschpuffer (PBS-Tween, wie oben angegeben) gewaschen.

4.4. Inkubation mit Detektor-Antikörper

Mit Peroxidase gekoppelter Detektor-Antikörper $K_s$ 19.2 wird in Puffer (150 mM NaCl, 10 mM Natriumphosphatpuffer pH 7,4, 1% Rinderserumalbumin) verdünnt (die optimale Konzentration liegt bei 0,2 - 0,5 U/ml), davon in jede Vertiefung der Mikrotiterplatte 100 $\times 10^{-6}$ l pipettiert und 90 min. bei Raumtemperatur inkubiert. Danach wird 2 x mit 200 $\times 10^{-6}$ l Waschpuffer (PBS-Tween, wie oben angegeben) und 4 x unter fließendem Wasser gewaschen.

4.5. Substratreaktion

Für eine Mikrotiterplatte wird 1 Substrattablette (10 mg) o-Phenylendiamin (Fa. Sigma) und 10 $\times 10^{-6}$ l 30% $H_2O_2$ entweder in 10 ml 0,1 M Kaliumphosphatpuffer (pH 6,0) oder in Citrat-Phosphat-Puffer (0,0347 M Citronensäure, 0,0667 M di-Natriumhydrogenphosphat; pH 5,0) gelöst (bei Verwendung von Citrat-Phosphat-Puffer erhält man höhere Absorptionen). In jede Vertiefung werden 100 $\times 10^{-6}$ l Substratlösung (auf Raumtemperatur temperiert) pipettiert. Die Mikrotiterplatte wird abgedeckt, um die Reaktion vor Lichteinwirkung zu schützen mit Alufolie o.ä.) und solange inkubiert (15-30 min.), bis sich eine entsprechende Farbintensität entwickelt hat.

4.6. Abstoppen der Enzymreaktion

Durch Zugabe von 50 $\times 10^{-6}$ l 12,5%iger $H_2SO_4$-Lösung wird die Reaktion der Peroxidase gestoppt. Bei quantitativen Bestimmungen ist zu beachten, daß für Standardprotein und Testserum nach der gleichen Zeit abgestoppt wird.

4.7. Auswertung

Die Mikrotiterplatten werden bei 492 nm in einem ELISA-Photometer durchgemessen.

BEISPIEL 5

Quantitative Bestimmung von Cytokeratin 8:18 im Sandwich-ELISA mit Mikrotiterplatten

5.1. Anstelle des Detektor-Antikörpers K$_s$ 19.2 wird der Peroxidase-gekoppelte Antikörper K$_s$ 18-27.IV oder K$_s$18-9B1 (gegen das Cytokeratin 18-Fragment gerichtet) eingesetzt. Sonst wie BEISPIEL 4.

5.2. Als Detektor wird der Peroxidase-gekoppelte Antikörper K$_s$ 8-17.2 (gegen das Cytokeratin 8-Fragment gerichtet) eingesetzt. Sonst wie BEISPIEL 4.

**Patentansprüche**

1. Verfahren zur Identifizierung des Differenzierungszustandes und des Ursprungs einer Zellgewebsprobe oder von Zellgewebstrümmern in Körperflüssigkeit durch Solubilisierung der in dieser Probe beziehungsweise Körperflüssigkeit vorhandenen unlöslichen Intermediärfilamentproteine in Form von löslichen Fragmenten, vorzugsweise der alpha-helikalen Mittelstücke, und Identifizierung dieser so erhaltenen oder bereits ohne zusätzliche Aufbereitung in der Probe enthaltenen Fragmente nach ihrem Ursprungsgewebetyp durch Immunreaktion mit spezifischen Antikörpern, wobei als Antikörper solche eingesetzt werden, die zur Identifizierung - vorzugsweise zur eindeutigen Identifizierung - von Intermediärfilamentproteinen nach ihrem Ursprungsgewebetyp geeignet sind und außerdem nach mehreren verschiedenen bekannten Testverfahren mit dem alpha-helikalen Mittelstück - beziehungsweise einem Epitop-tragenden Fragment davon - eines durch Rekonstitution aus gereinigten Monomeren des jeweils betreffenden Intermediärfilamentproteins gewonnenen Standards immunologisch reagieren, dadurch gekennzeichnet,

   daß ein Standard aus den zu identifizierenden Mittelstücken beziehungsweise Epitop-tragenden Fragmenten hergestellt wird, indem aus dem entsprechenden Gewebe oder einer geeigneten Zellkultur-Linie durch Extraktion und Reinigung die einzelnen Cytoskelett-Polypeptide isoliert werden,

   daß dann aus diesen Polypeptiden beziehungsweise im Falle der Cytokeratine aus einem äquimolaren Gemisch von Polypeptiden der relativ basischen (Typ II) und der sauren (Typ I) Unterfamilien als nächste Untereinheiten Dimere, sodann Tetramere und schließlich Protofilamente gebildet werden, und

   daß dann durch kontrollierte Verdauung mit Protease die alpha-helikalen Mittelstücke dieser Intermediärfilamente als dimere oder tetramere Komplexe freigesetzt, isoliert und gereinigt und als Standard eingesetzt werden.

2. Verfahren nach Anspruch 1, dadurch gekennzeichnet,

   daß die proteolytische Solubilisierung der zu identifizierenden Intermediärfilamentproteine bis maximal zur einfachen Teilung der alpha-helikalen Mittelstücke in jeweils zwei Mittelstückfragmente erfolgt.

3. Verfahren nach Anspruch 2, dadurch gekennzeichnet,

   daß die Solubilisierung mit Protease im Enzym-zu-Substrat-Verhältnis und einer Abbauzeit erfolgt, die derart bemessen sind, daß von allen zu identifizierenden Intermediärfilamentprotein-Typen die alpha-helikalen Mittelstücke beziehungsweise Epitop-tragende Fragmente davon freigesetzt sind.

4. Verfahren nach einem der vorhergehenden Ansprüche, dadurch gekennzeichnet,

   daß aus einer geeigneten Zellkultur-Linie durch kontrollierte Verdauung mit Protease aus dem Intermediärfilamentgerüst die alpha-helikalen Mittelstückfragmente freigesetzt, isoliert und gereinigt werden.

5. Verfahren nach einem oder mehreren der vorhergehenden Ansprüche, dadurch gekennzeichnet,

   daß als Antikörper eingesetzt werden:

   K$_s$ 8-17.2 zur Identifizierung eines alpha-helikalen Mittelstücks, von dem mindestens ein Monomer aus dem Cytokeratin No. 8 stammt,

   K$_s$ 18-27 IV und/oder K$_s$ 18-9B1 zur Identifizierung eines alpha-helikalen Mittelstücks, von dem mindestens ein Monomer aus dem Cytokeratin No. 18 stammt,

   K$_s$ 19.2 zur Identifizierung eines alpha-helikalen Mittelstücks, von dem mindestens ein Monomer aus dem Cytokeratin No. 19 stammt,

   K$_s$ pan 1-8 zur Identifizierung eines alpha-helikalen Mittelstücks, von dem mindestens ein oder mehrere Monomere aus einem oder mehreren der Cytokeratine No. 1 bis 8 stammen, und

18

VIM 3B4 zur Identifizierung eines alpha-helikalen Mittelstücks des Vimentins.

6. Verfahren nach einem oder mehreren der vorhergehenden Ansprüche, dadurch gekennzeichnet,

daß alpha-helikale Mittelstücke oder deren Epitoptragende Fragmente immunologisch durch Anwendung eines ELISA-Tests identifiziert werden,

daß dafür als an einer Mikro-Titerplatte zu fixierende Fänger-Antikörper solche Antikörper nach Anspruch 1 eingesetzt werden, die entweder aus Tierseren beziehungsweise als monoklonale Antikörper mittels der Hybridom-Technik gewonnen wurden und mit mehreren Epitopen beziehungsweise mit einem in mehreren Cytokeratinen vorkommenden Epitop reagieren,

daß als Detektor-Antikörper solche Antikörper nach Anspruch 1 eingesetzt werden, die eindeutig mit nur einem Intermediär-Filament-Polypeptid reagieren, und

daß die mit der zu untersuchenden Lösung gewonnenen Titerergebnisse mit Titerergebnissen verglichen werden, die mit einer eingestellten Lösung eines Standards gewonnen wurden, welcher Standard nach Anspruch 1 gewonnen wurde.

7. Verfahren nach Anspruch 6, dadurch gekennzeichnet,

daß als Fänger-Antikörper für Cytokeratine Antikörper $K_s$ pan 1-8 eingesetzt wird, der mit einem der Cytokeratine 1 bis 8 aus der Gruppe der Cytokeratine 1 bis 19 reagiert.

8. Verfahren nach Anspruch 6, dadurch gekennzeichnet,

daß als Detektor-Antikörper eingesetzt werden:

$K_s$ 8-7.2 für ds Cytokeratin No. 8,

$K_s$ 18-27 IV oder $K_s$ 18 = 9B1 für das Cytokeratin No. 18,

$K_s$ 19.2 für das Cytokeratin No. 19 und/oder

VIM 3B4 für Vimentin.

## Claims

1. Method for the identification of the differentiation state and the origin of a cellular tissue sample or cellular tissue fragments in body fluid by solubilizing the insoluble intermediate filament proteins in the form of soluble fragments and preferably alpha-helical central portions present in said sample or body fluid, and the identification of the portions obtained in this way or contained in the sample without any additional preparation in accordance with their origin tissue type by immune reaction with specific antibodies and the antibodies used are those which are suitable for identifying, preferably clearly identifying, intermediate filament proteins on the basis of their origin tissue type and also react according to several different, known test processes with the alpha-helical central portion, or an epitope-carrying fragment thereof, of a standard obtained by reconstitution from purified monomers of the particular intermediate filament protein, characterized in that a standard is prepared from the central portions or epitope-carrying fragments to be identified, in that the individual cytoskeleton polypeptides are isolated from the corresponding tissue or a suitable cell culture line by extraction and purification and that then from said polypeptides or in the case of cytokeratins from an equimolar mixture of polypeptides of the relatively basic (type II) and acid (type I) subfamilies as the next subunits are formed dimers, then tetramers and finally protofilaments and that then by controlled digestion with protease the alpha-helical central portions of these intermediate filaments are liberated as dimeric or tetrameric complexes, isolated, purified and used as a standard.

2. Method according to claim 1, characterized in that the proteolytic solubilization of the intermediate filament proteins to be identified takes place to maximum the simple division of the alpha-helical central fragments into in each case two central portion fragments.

3. Method according to claim 2, characterized in that the solubilization with the protease takes place in an enzyme-to-substrate ratio and degradation time, which are such that from all the intermediate filament protein types to be identified the alpha-helical central portions or epitope-carrying fragments thereof are liberated.

4. Method according to one of the preceding claims, characterized in that from a suitable cell culture line by controlled digestion with protease alpha-helical central portion fragments are liberated from the intermediate filament structure, isolated and purified.

**5.** Method according to one or more of the preceding claims, characterized in that as antibodies are used:

$K_s$ 8-17.2 for the identification of an alpha-helical central portion from which comes at least one monomer from the cytokeratin No. 8,

$K_s$ 18-27 IV and/or $K_s$ 18-9B1 for the identification of an alpha-helical central portion from which comes at least one monomer from the cytokeratin No. 18,

$K_s$ 19.2 for identifying an alpha-helical central portion which comes from at least one monomer from cytokeratin No. 19,

$K_s$ pan 1-8 for identifying an alpha-helical central portion from which come at least one or more monomers from one or more of the cytokeratins 1 to 8 and

VIM 3B4 for identifying an alpha-helical central portion of vimentin.

**6.** Method according to one or more of the preceding claims, characterized in that alpha-helical central portions or their epitope-carrying fragments are immunologically identified by using an ELISA test, that as catcher antibodies to be fixed to a microtitre plate those antibodies according to claim 1 are used, which have been either obtained from animal sera or as monoclonal antibodies by means of the hybridome process and react with several epitopes or with one epitope occurring in several cytokeratins, that as detector antibodies use is made of those antibodies according to claim 1, which clearly only react with a single intermediate filament polypeptide and that the titre results obtained with the solution under investigation are compared with titre results obtained with a set solution of a standard, which was obtained according to claim 1.

**7.** Process according to claim 6, characterized in that as catcher antibodies for cytokeratins use is made of antibodies $K_s$ pan 1-8, which reacts with one of the cytokeratins 1 to 8 from the group of cytokeratins 1 to 19.

**8.** Process according to claim 6, characterized in that as detector antibodies are used:

$K_s$ 8-17.2 for cytokeratin No. 8,

$K_s$ 18-27 IV or $K_s$ 18-9B1 for cytokeratin No. 18,

$K_s$ 19.2 for cytokeratin No. 19 and/or VIM 3B4 for vimentin.

## Revendications

**1.** Procédé d'identification de l'état de différenciation et de l'origine d'un échantillon de tissu de cellules ou de débris de tissus de cellules dans du fluide corporel,par solubilisation des protéines de filaments intermédiaires insolubles,présentes dans cet échantillon ou dans ce fluide corporel,sous forme de fragments solubles , avantageusement les pièces médianes d'hélice alpha, et identification de ces fragments ainsi obtenus,ou déjà contenus sans préparation supplémentaire dans l'échantillon,d'après leur type de tissu d'origine par réaction immunologique avec des anticorps spécifiques, où l'on utilise comme anticorps ceux qui conviennent bien pour l'identification - avantageusement pour une identification univoque- de protéines de filaments intermédiaires selon leur type de tissu d'origine,et qui,d'autre part,réagissent immunologiquement selon plusieurs procédés différents connus d'essai avec la pièce médiane d'hélice alpha - ou avec un fragment de cette pièce porteur d'épitope, d'un modèle étalon obtenu par reconstitution à partir de monomères purifiés de la protéine de filament intermédiaire en cause à chaque fois, procédé caractérisé en ce que

l'on prépare un modèle étalon à partir des pièces intermédiaires à identifier ou des fragments porteurs d'épitopes,en isolant les divers polypeptides de cytosquelette,par extraction et purification à partir des tissus correspondants ou d'une lignée convenable de culture de cellules,

l'on forme ensuite,à partir de ces polypeptides,ou dans le cas des cytokératines,à partir d'un mélange équimolaire depolypeptides des sous-familles relatives basiques (type II) ou acides ( type I)-,comme sous-unités les plus proches,des dimères, puis des tétramères et enfin des protofilaments,et

l'on met ensuite en liberté,par digestion réglée effectuée avec des protéases, les pièces intermédiaires d'hélice alpha de ces filaments intermédiaires,sous forme de complexes dimères ou tétramères,on les isole et les purifie et on les utilise comme modèle étalon .

**2.** Procédé selon la revendication 1, caractérisé en ce que la solubilisation protéolytique des protéines de filaments intermédiaires à identifier a lieu au maximum jusqu'à la séparation simple des pièces médianes d'hélice alpha dans chaque groupe de fragments de pièces médianes .

**3.** Procédé selon la revendication 2 , caractérisé en ce que la solubilisation à l'aide de protéase a lieu selon un rapport enzyme à substrat et en un temps de dégradation qui sont mesurés de façon à mettre en liberté,parmi tous les types de protéine de filaments intermédiaires à identifier,les pièces médianes d'hélice alpha ou les fragments porteurs d'épitopes.

**4.** Procédé selon l'une des revendications précédentes,caractérisé en ce qu'a partir d'une lignée convenable de culture de cellules,on met en liberté,par digestion réglée à l'aide de protéases,à partir du squelette de filaments intermédiaires,les fragments de pièce médiane d'hélice alpha,et l'on isole et purifie ces fragments .

**5.** Procédé selon une ou plusieurs des revendications précédentes, caractérisé en ce qu'on utilise comme anticorps:

$K_s$ 8-17.2 pour identifier une pièce médiane d'hélice alpha, d'où provient au moins un monomère de cytokératine n° 8,

$K_s$ 18-27 IV et/ou $K_s$ 18-9B1 pour identifier une pièce médiane d'hélice alpha d'où provient au moins un monomère de la cytokératine n° 18,

$K_s$ 19.2 pour identifier une pièce médiane d'hélice alpha, d'où provient au moins un monomère de la cytokératine n° 19,

$K_s$ pan 1-8 pour identifier une pièce médiane d'hélice alpha, d'où provien(nen)t un ou plusieurs monomères d'une ou plusieurs des cytokératines n° 1 à 8 ,et

VIM 3B4 pour identifier une pièce médiane d'hélice alpha de la vimentine .

**6.** Procédé selon une ou plusieurs des revendications précédentes, caractérisé en ce que

l'on identifie par voie immunologique,par application d'un essai ELISA ,des pièces médianes d'hélice alpha ou leurs fragments porteurs d'épitopes;

l'on utilise pour cela,comme anticorps capteur à fixer sur une plaque pour microtitrage, les anticorps selon la revendication 1 ,qui ont été obtenus à l'aide de la technique d'hybridome à partir de sérums d'animaux ou sous forme d'anticorps monoclonaux et qui réagissent avec plusieurs épitopes ou avec un épitope présent dans plusieurs cytokératines,

l'on utilise,comme anticorps détecteurs, les anticorps selon la revendication 1 ,qui réagissent de manière univoque avec seulement un polypeptide de filament intermédiaire, et l'on compare les résultats en titres obtenus pour la solution à examiner avec les résultats en titres qui ont été obtenus avec une solution ajustée d'un modèle étalon,lequel modèle a été obtenu selon la revendication 1 .

**7.** Procédé selon la revendication 6 , caractérisé en ce que l'on utilise ,comme anticorps capteur de cytokératines,un anticorps $K_s$ part 1-8,qui réagit avec une des cytokératines 1 à 8 du groupe des cytokératines 1 à 19 .

**8.** Procédé selon la revendication 6 ,caractérisé en ce qu'on utilise comme anticorps détecteur :

$K_s$ 8-17.2 pour la cytokératine n° 8 ,

$K_s$ 18-27 IV ou $K_s$ 18-9B1 pour la cytokératine n° 18,

$K_s$ 19.2 pour la cytokératine n° 19, et/ou

VIM 3B4 pour la vimentine .